# EUROPEAN PATENT APPLICATION

(11) **EP 4 456 381 A1**
(43) Date of publication of application: **30.10.2024**
(21) Application number: 24169209.4
(22) Date of filing: 09.04.2024
(51) Int. Cl.: H02K 1/34, H02K 33/16

(54) **LINEAR MOTOR AND MEDICAL CARE APPARATUS INCLUDING THE SAME**

(30) Priority: 24.04.2023 JP 2023070882
(71) Applicant: J. MORITA MANUFACTURING CORPORATION, Fushimi-ku, Kyoto-shi, Kyoto 6128533 (JP)
(72) Inventor: TANAKA, Tsuyoshi, Kyoto-shi, Kyoto, 612-8533 (JP)
(74) Representative: Müller Hoffmann & Partner

(57) **Abstract**

A linear motor (801) includes: a movable element (190) including a permanent magnet; a stator including a coil (52) positioned to face the permanent magnet; and a linear guide configured to guide the movable element (190) to move linearly. A support portion provided on the linear guide and a holding portion (160) provided on the movable element (190) to correspond to the support portion are fitted and thereby the movable element (190) is attached to the linear guide. The coil (52) includes a yoke (51) having a plate shape and a winding wire wound around the yoke (51). The winding wire is continuously wound around the yoke (51) to include a first region where the winding wire is wound around the yoke (51) in a first winding direction and a second region where the winding wire is wound around the yoke (51) in a second winding direction opposite to the first winding direction.

## Description

This application is based on Japanese Patent Application No. 2023-070882 filed on April 24, 2023 with the Japan Patent Office.

The present disclosure relates to a linear motor and a medical care apparatus including the same.

Conventionally, a three-dimensional scanner has been known as a medical care apparatus that scans a tooth and a surrounding soft tissue in an oral cavity and obtains three-dimensional shape data. For example, Japanese Patent No. 6883559 discloses a scanner in which a lens and a counterweight are moved linearly by a linear motor.

In the three-dimensional scanner disclosed in Japanese Patent No. 6883559, a plurality of support portions provided on the object side and holding portions provided on the plurality-of-linear-guides side, respectively, are fitted with play, which makes it possible to linearly move the lens and the counterweight by the linear motor while minimizing an influence of assembly errors of the apparatus, and obtain three-dimensional shape data of a tooth and a surrounding soft tissue in an oral cavity. However, in the linear motor disclosed in Japanese Patent No. 6883559, it is necessary to cause a current to flow through a coil in opposite directions before and after the lens in the linear movement direction, and an arc-shaped coil molded from a flat plate-like spiral coil is adopted. Therefore, the size of the coil itself is large, which results in an increase in size of the linear motor and a complicated structure. A linear motor in which different coils are arranged before and after a lens in a linear movement direction may be applicable. Such a linear motor, however, results in complicated control of the two coils and high manufacturing cost.

The present disclosure has been made to solve the above-described problem and an object thereof is to provide a linear motor that can be reduced in size, is easy to control, and can achieve low manufacturing cost, and a medical care apparatus including the liner motor.

To this end, a linear motor is provided that moves linearly, the linear motor including: a movable element including a permanent magnet; a stator including a coil positioned to face the permanent magnet; and a linear guide configured to guide the movable element to move linearly. A support portion provided on the linear guide and a holding portion provided on the movable element to correspond to the support portion are fitted and thereby the movable element is attached to the linear guide. The coil includes a yoke having a plate shape and a winding wire wound around the yoke. The winding wire is continuously wound around the yoke to include a first region where the winding wire is wound around the yoke in a first winding direction and a second region where the winding wire is wound around the yoke in a second winding direction opposite to the first winding direction.

A medical care apparatus is provided that includes: the linear motor described above; and a housing configured to hold the linear motor such that the movable element can move linearly along the linear guide.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

Fig. 1 is a schematic diagram showing a configuration of a three-dimensional scanner.
Fig. 2A is a schematic diagram showing a configuration of a handpiece, and Fig. 2B is a schematic diagram showing an X-Z cross section of the handpiece.
Fig. 3 is a diagram for illustrating a positional relationship between a lens and a counterweight in the three-dimensional scanner.
Fig. 4 is a perspective view of a linear motor.
Fig. 5 is an exploded perspective view of the linear motor.
Fig. 6 is a cross-sectional view of the linear motor taken along an X-Y cross section.
Fig. 7 is a perspective view of a coil.
Figs. 8A to 8C are cross-sectional views of the coil.
Fig. 9 is a perspective view of a yoke.
Fig. 10 is a diagram for illustrating a magnetic circuit of the linear motor.
Fig. 11 is a schematic diagram showing a configuration of a cutting apparatus that is a medical care apparatus according to a modification.

### DESCRIPTION

First, a configuration of a medical care apparatus of the present disclosure will be described. A three-dimensional scanner that can be used for dental care will be described as one example of the medical care apparatus. The three-dimensional scanner is an intra oral scanner for obtaining a three-dimensional shape of a tooth in an oral cavity. The three-dimensional scanner is not limited to the intra oral scanner, but is also applicable to any three-dimensional scanner having a similar configuration. For example, the three-dimensional scanner is also applicable to a scanner that takes an image of the inside of a human ear, in addition to the inside of an oral cavity, and obtains a three-dimensional shape of the inside of an outer ear. In addition, the medical care apparatus is not limited to the three-dimensional scanner, but is also applicable to a medical care apparatus in which a cutting tool is driven.

In addition, the medical care apparatus is applicable not only to a dental care but also to any type of medical cares in the fields of ophthalmology, otolaryngology, radiology, internal medicine, surgery, veterinary science, and the like. Also, the medical care includes a diagnosis and a treatment.

### [Configuration of Three-Dimensional Scanner]

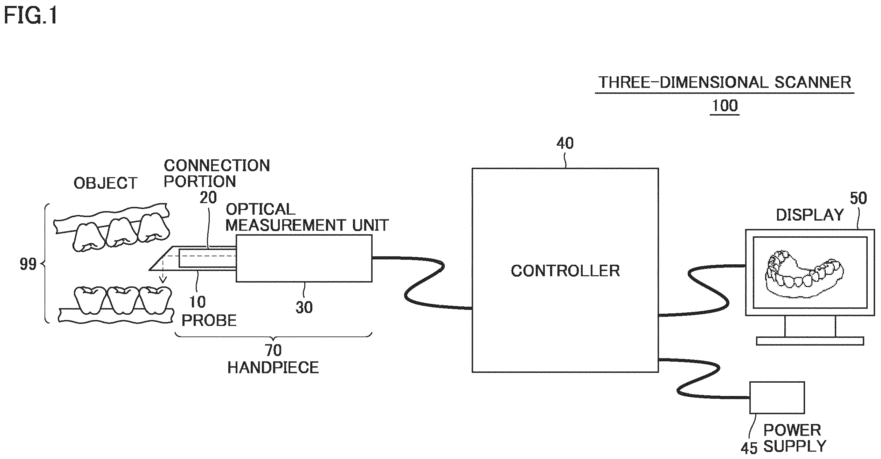

Fig. 1 is a schematic diagram showing a configuration of a three-dimensional scanner 100. Three-dimensional scanner 100 corresponds to one example of "medical care apparatus". As shown in Fig. 1, three-dimensional scanner 100 includes a handpiece 70, a controller 40, a display 50, and a power supply 45. Handpiece 70 is a hand-held member and includes a probe 10, a connection portion 20 and an optical measurement unit 30.

Probe 10 is inserted into an oral cavity and projects light having a pattern (hereinafter also simply referred to as "pattern") onto an object 99 such as a tooth. Probe 10 guides reflected light from object 99 having the pattern projected thereon to optical measurement unit 30. Probe 10 is removably mounted on connection portion 20 to cover an outer perimeter of a tip portion of connection portion 20. Therefore, an operator can remove only probe 10 that may come into contact with a living body from optical measurement unit 30 and perform sterilization treatment (e.g., autoclave treatment under a high-temperature and high-humidity environment) on probe 10 as infection countermeasures.

Connection portion 20 is a part of optical measurement unit 30, protrudes from optical measurement unit 30, and has a shape that can be fitted to the root of probe 10. Connection portion 20 includes an optical component such as a lens system for guiding the light taken in by probe 10 to optical measurement unit 30, a cover glass, an optical filter, and a phase difference plate (1/4 wavelength plate).

Optical measurement unit 30 projects the pattern onto object 99 through probe 10 and takes an image of the projected pattern. Although optical measurement unit 30 is configured to obtain a three-dimensional shape using the principle of a focusing method as described below, optical measurement unit 30 may be configured to obtain a three-dimensional shape using the principle such as the focusing method, a triangular method or a confocal method. In other words, optical measurement unit 30 may use any principle, as long as optical measurement unit 30 includes a configuration that changes a focal position of a projection pattern and an optical sensor and optical measurement unit 30 is configured to obtain a three-dimensional shape using an optical method.

Controller 40 controls the operation of optical measurement unit 30, and processes the image taken by optical measurement unit 30 and obtains a three-dimensional shape. Although not shown, controller 40 includes a central processing unit (CPU) serving as a control center, a read only memory (ROM) that stores a program, control data and the like for the operation of the CPU, a random access memory (RAM) that functions as a work area of the CPU, and an input/output interface for maintaining the signal integrity with a peripheral device. Controller 40 can also output the obtained three-dimensional shape to display 50, and can also input information such as setting of optical measurement unit 30 through a not-shown input device or the like.

At least a part of arithmetic operations for processing the taken image and obtaining the three-dimensional shape may be implemented as software by the CPU of controller 40, or may be implemented as hardware that performs processing separately from the CPU. At least a part of the processing unit such as the CPU or the hardware may be incorporated into optical measurement unit 30. Although Fig. 1 depicts each component (30, 40, 45, 50) of three-dimensional scanner 100 as being connected by a cable (a thick line in the figure), a part or the whole of the connection may be implemented by wireless communication. When controller 40 is small-sized and lightweight enough to lift controller 40 with one hand, controller 40 may be provided in handpiece 70.

Display 50 shows a measurement result of a three-dimensional shape of object 99 obtained by controller 40. Display 50 can also show other information such as setting information of optical measurement unit 30, patient information, an activation state of the scanner, a manual, and a help screen. A stationary liquid crystal display, a head-mounted display, a glass-type wearable display or the like is, for example, applicable as display 50. Alternatively, a plurality of displays 50 may be provided and the measurement result of the three-dimensional shape and the other information may be shown on the plurality of displays 50 simultaneously or in a divided manner.

Power supply 45 supplies electric power to optical measurement unit 30 and controller 40. Although power supply 45 may be provided outside controller 40 as shown in Fig. 1, power supply 45 may be provided inside controller 40 or inside handpiece 70. Alternatively, a plurality of power supplies 45 may be provided such that electric power can be supplied to controller 40, optical measurement unit 30 and display 50 individually.

### [Configuration of Handpiece]

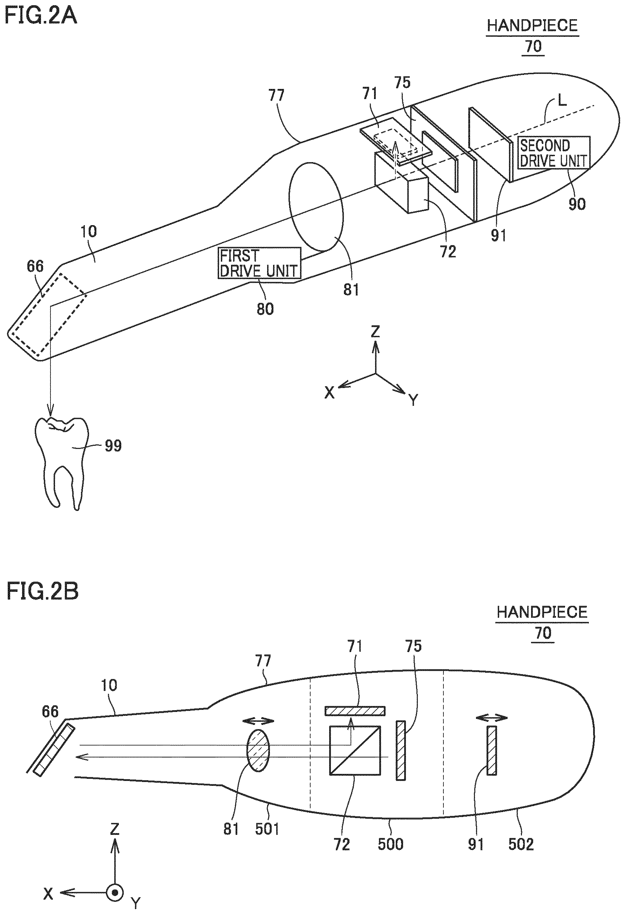

Fig. 2A is a schematic diagram showing a configuration of handpiece 70. Fig. 2B is a schematic diagram showing an X-Z cross section of handpiece 70. Each member in handpiece 70 shown in Figs. 2A and 2B is housed in optical measurement unit 30 shown in Fig. 1.

As shown in Figs. 2A and 2B, handpiece 70 includes a projection light generating unit 75, a lens 81, an optical sensor 71, and a prism 72 in a housing 77. In addition to these components, handpiece 70 may include a reflector that reflects light toward object 99, and the like. Lens 81 is held by a movable element of a linear motor described below. For convenience in description, in the below description, an imaginary straight line indicating a direction in which lens 81 reciprocates linearly is denoted by "L", an axis parallel to straight line L is referred to as "X axis (first axis)", an axis that is perpendicular to straight line L and faces upward on the sheet in Figs. 2A and 2B is referred to as "Z axis", and an axis perpendicular to each of the X axis and the Z axis is referred to as "Y axis (second axis)".

Projection light generating unit 75 is a laser element, a light emitting diode (LED) or the like serving as a light source. Light from projection light generating unit 75 passes through prism 72 and lens 81 via a projection pattern screen (not shown) arranged in front of projection light generating unit 75 to generate a projection pattern, and is emitted toward object 99 through a reflection unit 66 provided at probe 10, and is reflected by object 99. The light reflected by object 99 passes through lens 81 again via reflection unit 66 and enters prism 72. Prism 72 changes the traveling direction of the light from object 99 to a direction in which optical sensor 71 is located (in this example, a Z-axis direction). The light whose traveling direction has been changed by prism 72 is detected by optical sensor 71. In the example shown in Fig. 2B, the light from projection light generating unit 75 and the light reflected by object 99 and guided to prism 72 are shown separately. However, this is for making the description easier to understand, and actually, handpiece 70 is configured such that both of the lights are guided along the same axis.

When a three-dimensional shape is obtained using the technique of the focusing method, the light having passed through a pattern generating element (not shown) provided between lens 81 and object 99 is projected onto object 99. When lens 81 reciprocates linearly on the same straight line (e.g., straight line L shown in the figure), a focal position of the projection pattern changes. Optical sensor 71 detects the light from object 99 every time the focal position changes. Controller 40 described above computes shape information of object 99 based on the position of lens 81 and a result of detection by optical sensor 71 at that time.

Lens 81 is driven by a first drive unit 80 and reciprocates linearly. When lens 81 reciprocates linearly in the direction of straight line L (X-axis direction), the position of the center of gravity of handpiece 70 is moved by the mass of lens 81, which is transmitted to a hand of a user holding handpiece 70 in the form of vibration. In order to cancel the vibration, handpiece 70 further includes a counterweight 91 in housing 77. Counterweight 91 is driven by a second drive unit 90 and reciprocates linearly in a direction opposite to the direction of linear reciprocation of lens 81.

Counterweight 91 is provided on the rear surface side of projection light generating unit 75 in the X-axis direction so as not to block an optical path between object 99 and lens 81 and an optical path between lens 81 and optical sensor 71.

Specifically, as shown in Fig. 2B, handpiece 70 includes, in housing 77, a first accommodating portion 501 located in a front part of handpiece 70 and a second accommodating portion 502 located in a rear part of handpiece 70. Lens 81 is accommodated in first accommodating portion 501 and counterweight 91 is accommodated in second accommodating portion 502. Furthermore, between first accommodating portion 501 and second accommodating portion 502, handpiece 70 includes a coupling accommodating portion 500 that couples lens 81 accommodated in first accommodating portion 501 to counterweight 91 accommodated in second accommodating portion 502. Optical sensor 71, prism 72 and projection light generating unit 75 described above are accommodated in coupling accommodating portion 500.

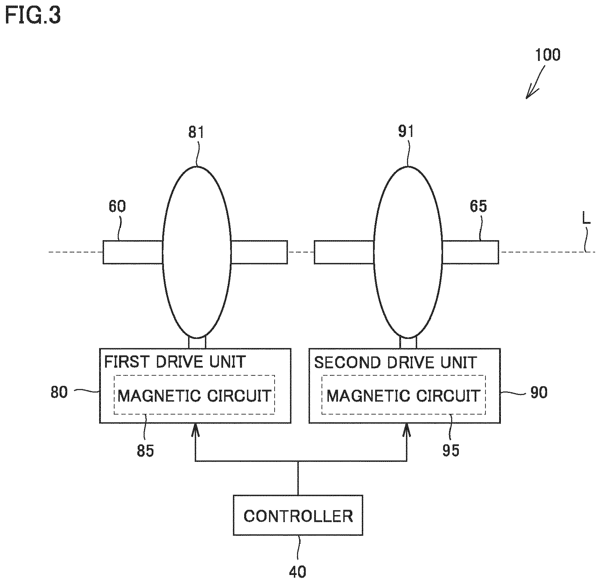

Fig. 3 is a schematic diagram for illustrating a positional relationship between lens 81 and counterweight 91 in three-dimensional scanner 100. In the example shown in Fig. 3, housing 77 is omitted. As shown in Fig. 3, lens 81 is supported to reciprocate linearly in the direction of straight line L by a linear guide 60 parallel to straight line L.

Furthermore, first drive unit 80 causes lens 81 held by the movable element to reciprocate linearly in the direction of straight line L by a magnetic circuit 85. In other words, first drive unit 80 is implemented by a linear motor.

Counterweight 91 is a weight provided on straight line L in the linear movement direction of lens 81 and having the same mass as that of lens 81. Counterweight 91 is supported to reciprocate linearly in the direction of straight line L by a linear guide 65 parallel to straight line L. Although linear guide 60 and linear guide 65 are described as different members, linear guide 60 and linear guide 65 may be formed by an integral member.

Furthermore, second drive unit 90 causes counterweight 91 held by the movable element to reciprocate linearly in the direction of straight line L by a magnetic circuit 95. In other words, second drive unit 90 is implemented by a linear motor.

A specific configuration of each of first drive unit 80 and second drive unit 90 that are linear motors will be described below. Hereinafter, first drive unit 80 and second drive unit 90 will also be collectively simply referred to as "linear motor". Each of first drive unit 80 and second drive unit 90 is controlled by controller 40. Although first drive unit 80 and second drive unit 90 are controlled by common controller 40, first drive unit 80 and second drive unit 90 may be controlled by different controllers.

When first drive unit 80 causes lens 81 to reciprocate linearly in the direction of straight line L serving as an optical axis, second drive unit 90 causes counterweight 91 to reciprocate linearly by the same distance as that of lens 81 in the direction opposite to the direction of linear reciprocation of lens 81. For example, when lens 81 moves on straight line L by 10 mm in the direction approaching object 99, counterweight 91 moves on straight line L by 10 mm in the direction going away from object 99. When lens 81 moves on straight line L by 15 mm in the direction going away from object 99, counterweight 91 moves on straight line L by 15 mm in the direction approaching object 99.

As described above, counterweight 91 reciprocates linearly by the same distance as that of lens 81 in the direction opposite to the direction of linear reciprocation of lens 81, and thus, the deviation of the center of gravity of handpiece 70 caused by the linear reciprocation of lens 81 can be canceled. As a result, the vibration caused by the linear reciprocation of lens 81 can be canceled by counterweight 91.

### [Configuration of Linear Motor]

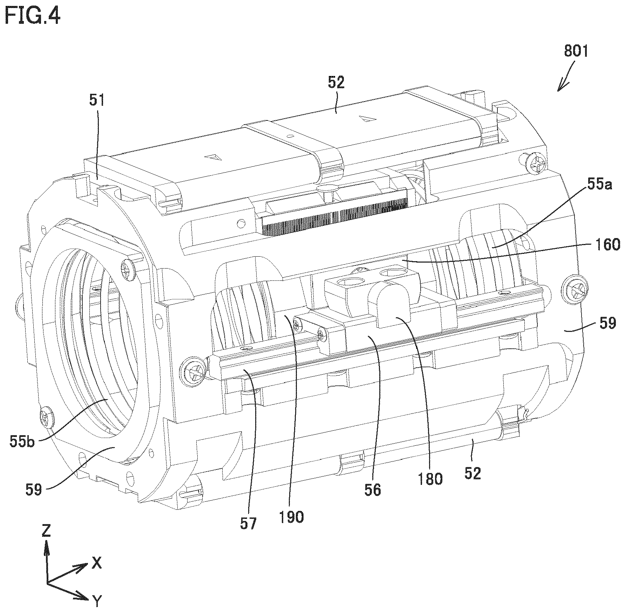

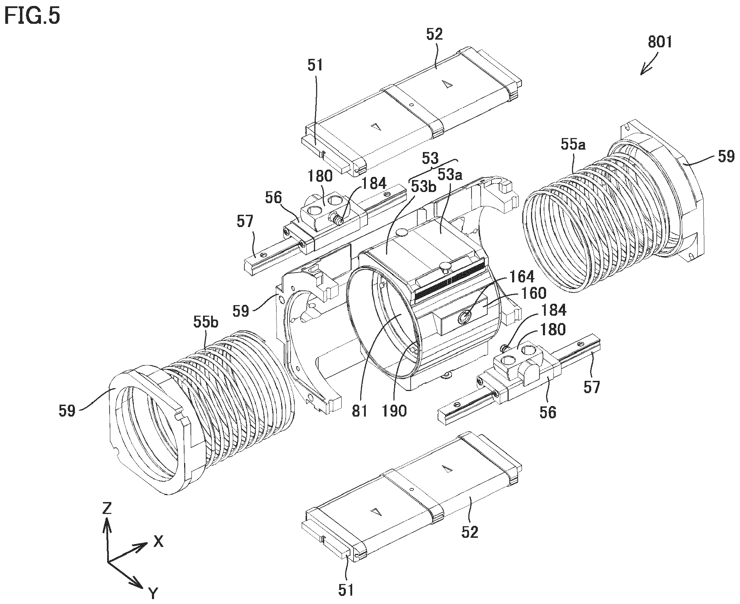

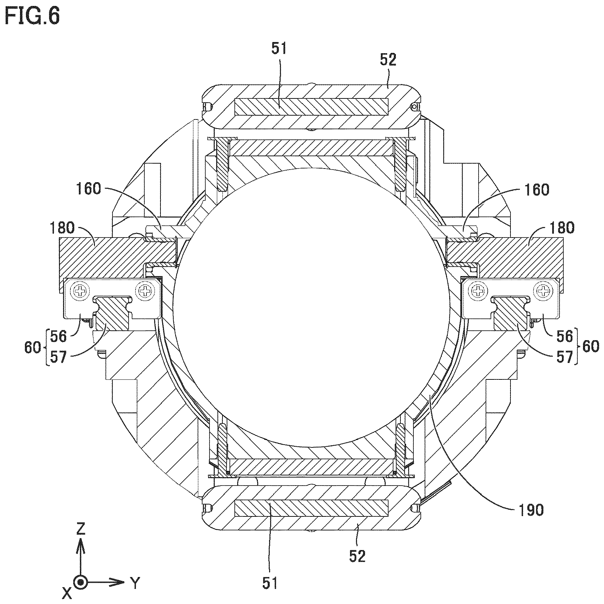

A specific configuration of the linear motor will now be described with reference to the drawings. Fig. 4 is a perspective view of a linear motor 801. Fig. 5 is an exploded perspective view of linear motor 801. Fig. 6 is a cross-sectional view of linear motor 801 taken along an X-Y cross section. Although a configuration of linear motor 801 corresponding to first drive unit 80, of the linear motor, is described in the example shown in Figs. 4 to 6, a configuration of the linear motor corresponding to second drive unit 90 is similar to the configuration of linear motor 801. Specifically, in the case of the linear motor corresponding to second drive unit 90, lens 81 is replaced with counterweight 91 in the example shown in Figs. 4 to 6, while the remaining configuration is the same as the configuration of linear motor 801.

Linear motor 801 has magnetic circuit 85 including a yoke 51, a coil 52 and a permanent magnet 53. In addition, linear motor 801 has a movable element 190 that holds lens 81 to surround an outer circumference of lens 81, and permanent magnet 53 is provided on each of the upper side and the lower side of movable element 190 in the figure. Portions of yoke 51 and coil 52 positioned to face permanent magnet 53 constitute a stator of linear motor 801. Permanent magnet 53 is arranged, for example, such that an N pole 53a thereof faces in the positive direction of the X axis and an S pole 53b thereof faces in the negative direction of the X axis, so as to fix movable element 190.

Since movable element 190 has an opening in the linear movement direction and holds lens 81 (optical component) in the opening, an optical path between object 99 and optical sensor 71 passes through the opening. A spring 55a abuts on one end of movable element 190 that holds lens 81, and a spring 55b abuts on the other end of movable element 190.

As shown in Fig. 6, in linear motor 801, two linear guides 60, each of which is made up of a rail 57 and a block 56, are provided in parallel with each other on an outer circumferential portion of movable element 190. Two linear guides 60 are arranged at different positions on the outer circumferential side of movable element 190.

More specifically, two linear guides 60 are arranged in parallel with each other at the positions where two linear guides 60 are rotationally symmetrical, with the optical axis (straight line L) that is parallel to the linear movement direction of lens 81 and passes through the center of lens 81 being a rotation axis.

Linear guide 60 includes block 56 and rail 57. Block 56 supports movable element 190 and lens 81 and is fitted onto rail 57, and movement of block 56 in the linear direction along rail 57 causes lens 81 to reciprocate linearly. A viscous lubricant such as grease may be applied to a connection surface between block 56 and rail 57, or the connection surface may be provided with a rolling bearing such as a ball or a roller.

Furthermore, as shown in Fig. 5, spring 55a and spring 55b are provided to surround the outer circumference of lens 81 so as not to block an optical path in the center portion of lens 81. Each of spring 55a and spring 55b corresponds to one example of an elastic member. A coil spring or the like is applied as each of spring 55a and spring 55b. The elastic member is not limited to the spring, but may be any member such as rubber as long as the member is deformed when the force is applied and returns to the original state when the force is removed.

One end of each of spring 55a and spring 55b abuts on movable element 190 and the other end is fixed by a case 59 of linear motor 801. Furthermore, spring 55a and spring 55b are held in case 59 such that spring 55a and spring 55b are allowed to be deformed in the X direction and are difficult to be deformed in the Y-Z direction. Spring 55a and spring 55b arranged as described above provide the elastic force to movable element 190 in the linear movement direction. It is preferable that a diameter of each of spring 55a and spring 55b should be substantially the same as a diameter of lens 81 so as not to block the optical path passing through lens 81.

Lens 81 is supported to be capable of reciprocating linearly by movable element 190 and also by linear guides 60 via support portions 180 and holding portions 160. Specifically, each of holding portions 160 is provided on a part of movable element 190 that holds lens 81. Each of support portions 180 is screwed onto a part of block 56 that moves on rail 57. Support portion 180 and holding portion 160 are fitted.

As described above, support portion 180 screwed on the linear guide 60 side and holding portion 160 provided on the movable element 190 side are fitted, whereby movable element 190 including lens 81 can reciprocate linearly along rail 57.

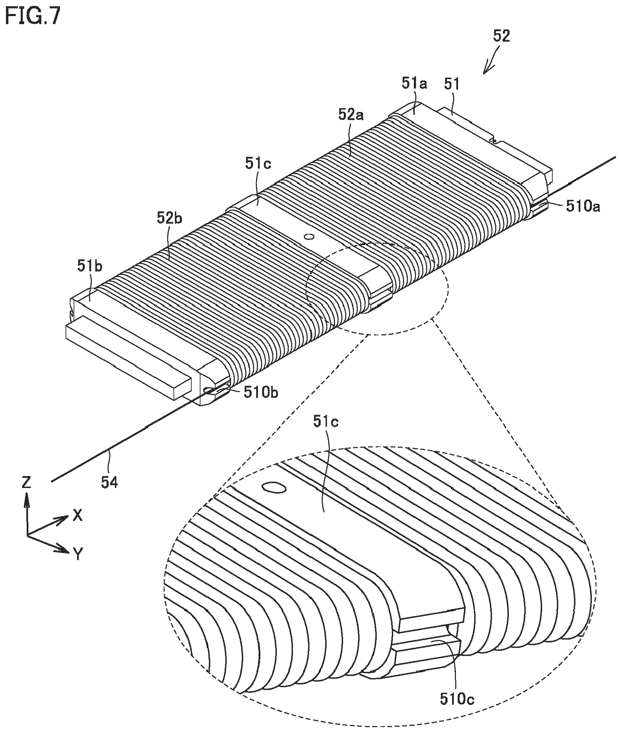

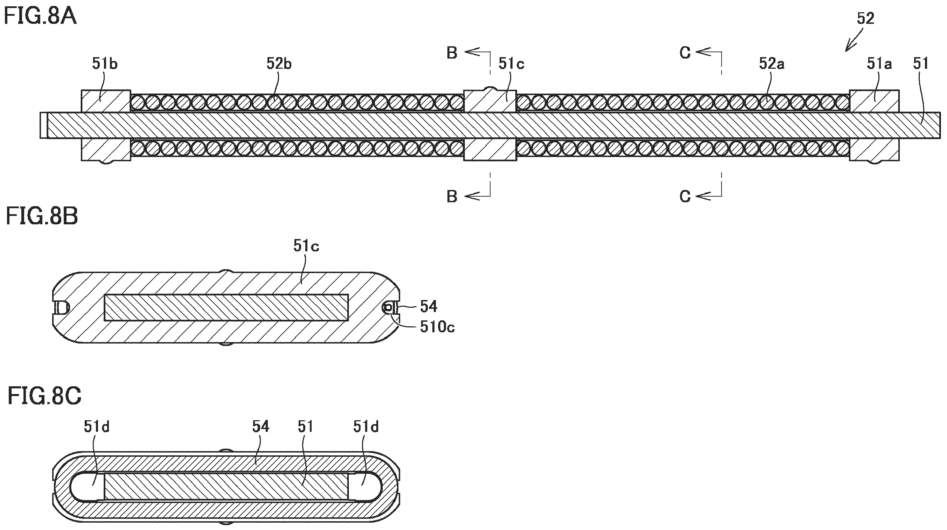

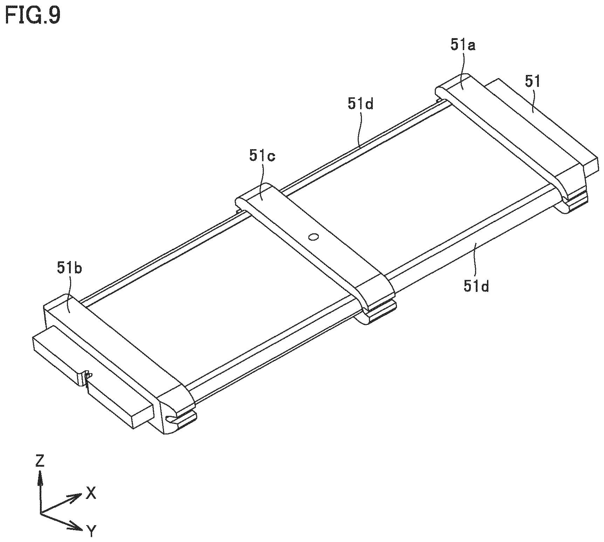

Coil 52 is not a flat plate-like spiral coil, but is a solenoid coil formed by winding a winding wire around a yoke having a plate shape. Specifically, Fig. 7 is a perspective view of such a coil 52. Figs. 8A to 8C are cross-sectional views of coil 52. Fig. 9 is a perspective view of yoke 51. As shown in Fig. 7, coil 52 includes a first region 52a where a winding wire 54 is wound around yoke 51 in the counterclockwise direction (first winding direction) when facing in the negative-direction of the X axis, and a second region 52b where winding wire 54 is wound around yoke 51 in the clockwise direction (second winding direction) when facing in the negative-direction of the X axis. Winding wire 54 starts to be wound from a notch portion 510a of a resin portion 51a, is continuously wound in first region 52a and second region 52b, and ends at a notch portion 510b of a resin portion 51b. Since the winding direction of winding wire 54 in first region 52a is different from the winding direction of winding wire 54 in second region 52b, the winding direction of winding wire 54 is switched at a notch portion 510c of a resin portion 51c.

As shown in Fig. 8A, in coil 52, winding wire 54 is wound on both sides of yoke 51. Although the configuration in which one layer of winding wire 54 is wound around yoke 51 has been described, two or more layers of winding wire 54 may be wound around yoke 51. As shown in Fig. 8B, in resin portion 51c, winding wire 54 exists only in notch portion 510c and winding wire 54 does not exist in the other portion. As shown in Fig. 8C, in the portions where resin portions 51a to 51c are not provided, winding wire 54 exists so as to surround yoke 51. A resin portion 51d is provided on a side surface of yoke 51 to prevent wound winding wire 54 from being damaged by a corner of yoke 51.

Yoke 51 is formed by integrally molding resin portions 51a to 51d, by outsert molding, onto a metal plate (such as, for example, permalloy, silicon steel plate or low carbon steel) having a plate shape as shown in Fig. 9. In other words, resin portions 51a to 51d function as bobbins of coil 52, and the yoke and the bobbins can be integrally molded by outsert molding of resin portions 51a to 51d onto yoke 51. As a matter of course, resin portions 51a to 51d may be molded separately and resin portions 51a to 51d may be individually attached to yoke 51. A region between resin portion 51a and resin portion 51c corresponds to first region 52a, and a region between resin portion 51b and resin portion 51c corresponds to second region 52b. When lens 81 reciprocates linearly from the center position as in the three-dimensional scanner, it is preferable that first region 52a and second region 52b should have the same area. As a matter of course, first region 52a and second region 52b may have different areas.

Furthermore, although coil 52 includes resin portion 51c and switching between the winding direction of winding wire 54 in first region 52a and the winding direction of winding wire 54 in second region 52b is done in resin portion 51c, the winding end of winding wire 54 may be fixed at the position where the winding direction is switched, without providing resin portion 51c. Although a width (a length in the X-axis direction) of resin portion 51c may be appropriately determined depending on an object to be driven, making the width of resin portion 51c smaller leads to a further reduction in size of coil 52. Although the winding start of winding wire 54 has been described as resin portion 51a and the winding end has been described as resin portion 51b, it may be the other way around, and the winding end of winding wire 54 may be fixed at the winding start position and the winding end position, without providing resin portions 51a and 51b.

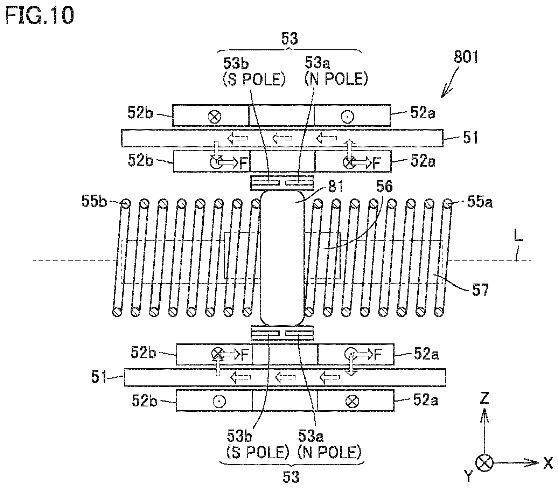

A magnetic circuit when solenoid coil-type coil 52 is adopted in linear motor 801 will be described. Fig. 10 is a diagram for illustrating a magnetic circuit of linear motor 801. When permanent magnet 53 is arranged such that N pole 53a and S pole 53b are located with respect to coil 52 with a positional relationship shown in Fig. 10, a magnetic field occurs in a direction indicated by a dashed arrow. In coil 52, a current shown in Fig. 10 (the current from front to back on the sheet along the Y axis is indicated by "×" and the current from back to front on the sheet along the Y axis is indicated by "·") is passed through each of first region 52a and second region 52b of coil 52.

When the current is passed through coil 52, the electromagnetic force (F) is generated in a direction indicated by a solid arrow (X-axis direction) in accordance with the Fleming's left hand rule. When the reaction force of the electromagnetic force (F) thus generated acts on permanent magnet 53 provided on movable element 190, lens 81 moves in the direction opposite to the electromagnetic force (F). Lens 81 can be moved in the opposite direction by reversing the direction of the current flowing through coil 52. Therefore, in linear motor 801, the linear reciprocation of lens 81 can be achieved by periodically changing the direction of the current flowing through coil 52.

As shown in Fig. 10, the current flowing on the side of coil 52 that faces permanent magnet 53 with yoke 51 being interposed contributes to the electromagnetic force (F), whereas the current flowing on the opposite side does not contribute to the electromagnetic force (F). In addition, one winding wire 54 forms first region 52a and second region 52b of coil 52 that are different in the winding direction. Therefore, simply by passing the current through winding wire 54, currents having the same phase and having opposite directions on the magnetic circuit can be passed through first region 52a and second region 52b of coil 52. If different winding wires form a coil of first region 52a and a coil of second region 52b, control of the two coils becomes complicated and the manufacturing cost becomes high. However, since one winding wire 54 forms first region 52a and second region 52b of coil 52, such a problem does not occur.

### [Modifications]

The present disclosure is not limited to the above description, and various modifications and applications are possible. A modification applicable to the present disclosure will be described below.

The above-described configuration of linear motor 801 is one example, and the number of springs and the locations where the springs are arranged, and further, the number of linear guides and the locations where the linear guides are arranged can be combined as appropriate and designed in consideration of the space in the handpiece. In addition, although linear motor 801 includes two combinations of permanent magnet 53 and coil 52 positioned to face permanent magnet 53 on the upper side and on the lower side of movable element 190 as shown in Fig. 5, linear motor 801 may include any one of these combinations, or may include three or more combinations.

Although in the above description support portion 180 having protruding portion 184 protruding toward movable element 190 is provided on the linear guide 60 side and holding portion 160 having recessed portion 164 fitted onto protruding portion 184 is provided on the movable element 190 side, support portion 180 may be provided on the movable element 190 side and holding portion 160 may be provided on the linear guide 60 side. In addition, the shape of each of protruding portion 184 and recessed portion 164 is not limited to the cylindrical shape, but may be any shape. For example, protruding portion 184 may have a spherical shape protruding outward toward the movable element 190 side and recessed portion 164 may have a spherical recess.

Although the three-dimensional scanner is described as one example of the medical care apparatus, the medical care apparatus is also applicable to other uses. Examples of the medical care apparatus include a cutting apparatus that cuts or grinds away an object using a cutting tool (e.g., a scaler tip, a file for root canal treatment, or the like).

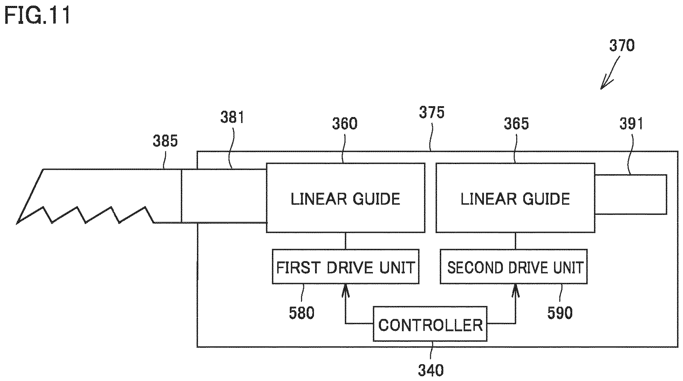

Fig. 11 is a schematic diagram showing a configuration of a cutting apparatus 370 that is a medical care apparatus according to a modification. As shown in Fig. 11, cutting apparatus 370 includes a case 375, a cutting tool 385, a cutting holding unit 381 that holds cutting tool 385, a first drive unit 580 that causes cutting holding unit 381 to reciprocate linearly, a counterweight 391 provided on a straight line in a linear movement direction of cutting holding unit 381 and having the same mass as that of cutting holding unit 381, a second drive unit 590 that causes counterweight 391 to reciprocate linearly, a linear guide 360 that guides cutting holding unit 381 such that cutting holding unit 381 reciprocates linearly, a linear guide 365 that guides counterweight 391 such that counterweight 391 reciprocates linearly, and a controller 340 that controls first drive unit 580 and second drive unit 590. Counterweight 391 may have the same mass as a total of the mass of cutting holding unit 381 and the mass of cutting tool 385. Controller 340 controls each of first drive unit 580 and second drive unit 590 such that cutting holding unit 381 and counterweight 391 reciprocate linearly by the same distance in opposite directions.

As described above, in cutting apparatus 370, controller 340 independently controls first drive unit 580 that causes cutting holding unit 381 to reciprocate linearly and second drive unit 590 that causes counterweight 391 to reciprocate linearly, whereby an object (e.g., a tooth) can be cut or ground away using cutting tool 385 held by cutting holding unit 381 and a residual vibration caused by the linear reciprocation of cutting holding unit 381 can be suppressed as much as possible by counterweight 391.

Although controller 340 is housed in case 375 in cutting apparatus 370 shown in Fig. 11, controller 340 may be arranged outside case 375 and connected to first drive unit 580 and second drive unit 590 by a wire as in three-dimensional scanner 100 shown in Fig. 1.

In addition, a medical camera that takes an image of the inside of an oral cavity, the inside of an outer ear, or a digestive organ such as stomach or intestine may be applied as the medical care apparatus. In this case, a lens of the camera may be applied as an object held by the movable element of the linear motor, and a counterweight may be applied as another movable element.

In addition, a microscope may be applied as the medical care apparatus. In this case, a lens in the microscope may be applied as an object held by the movable element of the linear motor, and a counterweight may be applied as another movable element.

Furthermore, a laser pointer that points to an object such as a chart using a laser beam or a laser apparatus that grinds a tooth may be applied as the medical care apparatus. In this case, a lens may be applied as an object held by the movable element of the liner motor, and a counterweight may be applied as another movable element.

It should be understood that the above description is illustrative and non-restrictive in every respect. The present invention is defined by the claimed subject-matter, rather than the description above, and is intended to include any modifications within the claimed subject-matter. It is to be noted that the above described configurations can be combined as appropriate.

## Claims

1. A linear motor (801) that is configured to move linearly, the linear motor (801) comprising:
a movable element (190) including a permanent magnet (53);
a stator including a coil (52) positioned to face the permanent magnet (53); and
a linear guide (60, 65) configured to guide the movable element (190) to move linearly, wherein
a support portion (180) provided on the linear guide (60, 65) and a holding portion (160) provided on the movable element (190) to correspond to the support portion (180) are fitted and thereby the movable element (190) is attached to the linear guide (60, 65),
the coil (52) includes a yoke (51) having a plate shape and a winding wire (54) wound around the yoke (51), and
the winding wire (54) is continuously wound around the yoke (51) to include a first region (52a) where the winding wire (54) is wound around the yoke (51) in a first winding direction and a second region (52b) where the winding wire (54) is wound around the yoke (51) in a second winding direction opposite to the first winding direction.

2. The linear motor (801) according to claim 1, wherein
a first resin portion (51a) located at a winding start of the first region (52a), a second resin portion (51b) located at a winding end of the second region (52b), and a third resin portion (51c) located at a boundary between the first region (52a) and the second region (52b) are integrally molded on the yoke (51) by outsert molding.

3. The linear motor (801) according to claim 2, wherein
switching between the first winding direction and the second winding direction is done in the third resin portion (51c) and the winding wire (54) is continuously wound around the yoke (51).

4. The linear motor (801) according to any one of claims 1 to 3, wherein
the first region (52a) and the second region (52b) have the same area.

5. The linear motor (801) according to any one of claims 1 to 4, wherein
the support portion (180) includes a protruding portion (184) protruding outward toward the movable element (190) side, and
the holding portion (160) includes a recessed portion (164) fitted onto the protruding portion (184).

6. The linear motor (801) according to claim 5, wherein
the protruding portion (184) has a cylindrical or spherical shape protruding outward toward the movable element (190) side, and
the recessed portion (164) has a shape that allows the recessed portion (164) to be fitted onto the protruding portion (184) having the cylindrical or spherical shape.

7. The linear motor (801) according to any one of claims 1 to 6, wherein
the linear motor (801) includes a plurality of combinations of the permanent magnet (53) and the coil (52) positioned to face the permanent magnet (53).

8. The linear motor (801) according to any one of claims 1 to 7, wherein
the movable element (190) includes an elastic member (55a, 55b) on at least one of surfaces perpendicular to a linear movement direction.

9. The linear motor (801) according to any one of claims 1 to 8, wherein
the linear guide (60, 65) includes two linear guides (60, 65) provided in parallel with each other to guide the movable element (190) to move linearly.

10. The linear motor (801) according to any one of claims 1 to 9, wherein
the movable element (190) has an opening in a linear movement direction and can hold an optical component (81) in the opening.

11. The linear motor (801) according to any one of claims 1 to 9, wherein
the movable element (190) includes a cutting holding unit (381) configured to hold a cutting tool.

12. A medical care apparatus comprising:
the linear motor (801) as recited in any one of claims 1 to 11; and
a housing configured to hold the linear motor (801) such that the movable element (190) can move linearly along the linear guide (60, 65).

13. The medical care apparatus according to claim 12, wherein
the medical care apparatus is a hand-held three-dimensional scanner (100).
